(19) Europäisches Patentamt
European Patent Office
Office européen des brevets

(11) Veröffentlichungsnummer : **0 352 489 B1**

(12) **EUROPÄISCHE PATENTSCHRIFT**

(45) Veröffentlichungstag der Patentschrift : **17.06.92 Patentblatt 92/25**

(51) Int. Cl.$^5$ : **A61K 7/13,** C09B 51/00

(21) Anmeldenummer : **89111641.0**

(22) Anmeldetag : **27.06.89**

# (54) Verwendung von 2,6-Dinitro-phenol-derivaten in Haarfärbemitteln.

(30) Priorität : **23.07.88 DE 3825163**

(43) Veröffentlichungstag der Anmeldung : **31.01.90 Patentblatt 90/05**

(45) Bekanntmachung des Hinweises auf die Patenterteilung : **17.06.92 Patentblatt 92/25**

(84) Benannte Vertragsstaaten : **AT BE CH DE ES FR GB GR IT LI NL SE**

(56) Entgegenhaltungen :
**EP-A- 0 234 305**
**US-A- 3 344 031**
**CANADIAN JOURNAL OF CHEMISTRY, Band 31, 1953, Seiten 837-841, The National Research Council, Ottawa, CA; G. DUTTON et al.:"Preparation of dinitro-n-alkyl phenols"**

(73) Patentinhaber : **Wella Aktiengesellschaft**
**Berliner Allee 65**
**W-6100 Darmstadt (DE)**

(72) Erfinder : **Clausen, Thomas, Dr.**
**Ernst-Pasqué-Strasse 35 a**
**W-6146 Alsbach (DE)**
Erfinder : **Balzer, Wolfgang, Dr.**
**Im Kiesling 12**
**W-6146 Alsbach (DE)**
Erfinder : **Lang, Günther, Dr.**
**Auf der roten Erde 10 b**
**W-6107 Reinheim 5 (DE)**

EP 0 352 489 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Gegenstand der Erfindung ist die Verwendung von Nitrofarbstoffen zum Färben von Haaren, wobei als Nitrofarbstoffe bestimmte 2,6-Dinitro-phenolderivate verwendet werden, sowie Mittel mit einem Gehalt an 2,6-Dinitro-phenolderivaten.

Nitrofarbstoffe finden heute in Haarfärbemitteln eine breite Anwendung. Sie werden in Oxidationshaarfärbemitteln als Zusätze für die Erzeugung von natürlichen oder modischen Farbnuancen verwendet. Durch Kombination von mehreren verschieden gefärbten Nitrofarbstoffen ist es jedoch auch möglich, Haarfärbemittel herzustellen, die Haare in natürlichen bis modischen Nuancen ohne die Anwendung von Oxidationsmitteln zu färben vermögen.

So können zum Beispiel durch die Kombination eines orangefärbenden mit einem blaufärbenden Nitrofarbstoff natürlich wirkende, braune Färbungen erzeugt werden. Daneben ist es jedoch auch möglich, mit einem gelbfärbenden und einem violettfärbenden Nitrofarbstoff ein ähnliches Ergebnis zu erhalten. Es werden daher gelbe Nitrofarbstoffe benötigt, die das Haar entweder in einem intensiven, reinen Zitronengelb zu färben vermögen, welches möglichst frei von Rotanteilen sein muß, oder solche, die orange färben und in Kombination mit rein blauen Farbstoffen eingesetzt werden können.

Daneben werden an Haarfarbstoffe noch viele weitere Anforderungen gestellt. Die Nitrofarbstoffe müssen in toxikologischer und dermatologischer Hinsicht unbedenklich sein.

Ihre Verwendung in Oxidationshaarfärbemitteln setzt voraus, daß sie in Anwesenheit von Wasserstoffperoxid in alkalischer Lösung stabil sind. Außerdem wird für die erzeugten Haarfärbungen eine gute Licht-, Säure-, und Reibechtheit gefordert. Schließlich sollten die Nitroverbindungen durch möglichst einfache Verfahren herstellbar sein.

Die bisher in der Literatur beschriebenen substituierten Nitrophenole und Aminonitrophenole erfüllen die vorstehend genannten Voraussetzungen nicht oder nur unzureichend. So läßt sich mit substituierten 2-Nitrophenolen keinerlei Anfärbung von Haaren erreichen. Das im International Journal of Cosmetic Science 1982, Seiten 25 - 35 genannte 4-Nitro-3-(2′-hydroxyethyl)amino-phenol ergibt zwar eine zitronengelbe Färbung, diese ist jedoch sehr farbschwach. Zwei weitere Isomere, nämlich das 4-Nitro- und das 5-Nitro-2-(2′-hydroxyethyl)amino-phenol, sind pH-empfindlich und zeigen unerwünschte Farbänderungen bei Säure- oder Alkalieinwirkungen.

Weitere bekannte gelbe Nitrofarbstoffe sind die in der DE-AS 1 619 395 genannten o-, m- und p-Nitroanilinderivate. Diese Verbindungen erfüllen zwar weitgehend die Anforderungen in anwendungstechnischer Hinsicht, befriedigen aber nicht im Hinblick auf die physiologischen Eigenschaften.

Die in der DE-OS 3 442 757 beschriebenen 2-Nitro-anilinderivate erfüllen zwar weitgehend die gestellten Anforderungen, jedoch läßt sich die durch Variation der Substituenten erzielbare Farbskala nicht bis in den Rotbereich hin ausdehnen. Deshalb können mit den dort beschriebenen 2-Nitro-anilinderivaten ohne die zusätzliche Anwendung von Rotfarbstoffen keine modischen rötlichen Haarfarben hergestellt werden.

Zudem besteht eine Schwierigkeit bei der Haarfärbung mit Farbstoffgemischen in der Erzielung einer gleichmäßigen Anfärbung über die gesamte Länge der Haare, da diese in der Regel in den Spitzen geschädigt sind und somit dort eine porösere Oberfläche besitzen als an der Haarwurzel.

Eine gleichmäßige Färbung läßt sich erreichen, indem Farbstoffe von gleicher Grundstruktur verwendet werden, die sich in ihrer Selektivität und in ihrem Aufziehvermögen entsprechen.

Weiterhin besteht bei der Verwendung von Farbstoffen gleicher Struktur der Vorteil, daß sich diese Farbstoffe auch in bezug auf die Lichtechtheit und Auswaschbarkeit gleich verhalten. Damit werden zum Beispiel Farbverschiebungen durch die bevorzugten Auswaschungen eines Farbstoffes aus einem Farbstoffgemisch durch wiederholtes Shampoonieren vermieden.

Es bestand demnach die Aufgabe, Nitrofarbstoffe für die Verwendung in Haarfärbemitteln zur Verfügung zu stellen, welche die vorteilhaften Eigenschaften der Verbindungen gemäß der DE-OS 3 442 757 aufweisen und darüber hinaus eine Ausweitung der Farbskala (durch Variation der Substituenten) in den Rotbereich ermöglichen.

Es wurde nun gefunden, daß durch die Verwendung eines 2,6-Dinitro-phenolderivats der allgemeinen Formel (I)

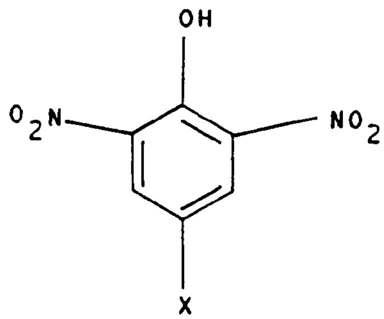

(I),

wobei X einen der Reste ($C_1$-$C_4$)-Alkyl, ($C_1$-$C_4$)-Monohydroxyalkyl, ($C_1$-$C_4$)-Perfluoralkyl, ($C_1$-$C_4$)-Alkoxy, ($C_2$-$C_4$)-Monohydroxyalkoxy, ($C_3$-$C_4$)-Dihydroxyalkoxy oder Halogen darstellt, als Farbstoff in Haarfärbemitteln die gestellte Aufgabe in hervorragender Weise gelöst wird.

Durch Variation des Restes X stehen Verbindungen der Formel (I) zur Verfügung, die nunmehr alle benötigten Farbtöne, von einem blaustichigen Zitronengelb über ein reines Gelb bis zu einem Orangerot liefern.

Im Vergleich zu den Farbstoffen des 2-Nitro-anilin-Typs gemäß der DE-OS 3 442 757 läßt sich mit den Verbindungen der allgemeinen Formel (I) die Farbskala bis in den Rotbereich hinein ausdehnen. Hierdurch ist es möglich, durch Kombination dieser Farbstoffe mit geeigneten blauen oder violetten Farbstoffen nicht nur natürlich wirkende, braune Färbungen zu erzeugen, sondern ohne die zusätzliche Anwendung von Rotfarbstoffen mit anderer Grundstruktur auch modische, rötliche Haarfarben herzustellen.

Von den unter die Formel (I) fallenden Verbindungen ist aus färberischen und physiologischen Gründen die Verwendung von 2,6-Dinitro-phenolderivaten der allgemeinen Formel (II)

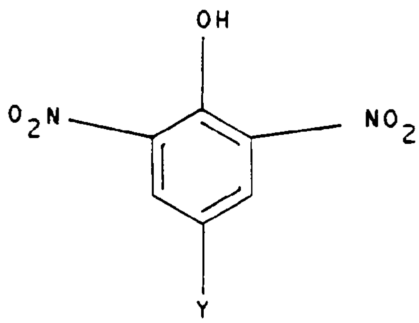

(II),

wobei Y einen der Reste $CH_3$, $C(CH_3)_3$, $CF_3$, $CH_2OH$, $OCH_3$, Cl oder Br darstellt, als Farbstoff in Haarfärbemitteln bevorzugt.

Beispiele für erfindungsgemäß geeignete 2,6-Dinitro-phenolderivate der allgemeinen Formel (I) sind 2,6-Dinitro-4-methyl-phenol, 2,6-Dinitro-4-trifluormethyl-phenol, 2,6-Dinitro-4-methoxy-phenol, 4-Chlor-2,6-dinitro-phenol und 4-Brom-2,6-dinitro-phenol.

Die Verbindungen der allgemeinen Formel (I) stellen für die Färbung von menschlichen Haaren hervorragend geeignete, gelb bis rotorange färbende Nitrofarbstoffe dar. Sie sind gut in Wasser löslich und weisen eine ausgezeichnete Lagerstabilität auf.

Überraschend sind die guten physiologischen Eigenschaften der Verbindungen der allgemeinen Formel (I). So zeigen zum Beispiel die getesteten Dinitrophenole mit X = $C(CH_3)_3$, $CF_3$ und Br im Amestest keine mutagene Wirkung.

Gegenstand der vorliegenden Anmeldung ist daher ebenfalls ein Mittel zur Färbung von Haaren mit einem Farbstoffgehalt und für Haarfärbemittel üblichen kosmetischen Zusätzen, dadurch gekennzeichnet, daß es ein 2,6-Dinitro-phenolderivat der allgemeinen Formel (I), vorzugsweise in einer Menge von 0,01 bis 2,0 Gewichtsprozent, enthält.

Das erfindungsgemäße Mittel zur Färbung von Haaren betrifft sowohl eine Ausführungsform, bei der es ohne Zugabe eines Oxidationsmittels angewendet wird, als auch eine weitere Ausführungsform, bei der die Zugabe eines Oxidationsmittels erforderlich ist.

Bei dem ersteren Haarfärbemittel ohne Oxidationsmittelzugabe handelt es sich um ein Mittel, das neben den Farbstoffen der angegebenen Formel (I) noch andere bekannte direkt auf das Haar aufziehende Farbstoffe enthalten kann. Von diesen für die Haarfärbung bekannten Farbstoffen seien beispielsweise die folgenden

erwähnt: aromatische Nitrofarbstoffe, wie z. B. 2-Amino-4-nitro-phenol, Pikraminsäure, 2-(2′-Hydroxyethyl)amino-4,6-dinitro-benzol, 1-(2′-Hydroxyethyl)amino-2-amino-4-nitro-benzol, 4-(2′-Ureidoethyl)amino-nitrobenzol, 4-(2′,3′-Dihydroxypropyl)amino-3-nitro-trifluormethylbenzol, 1,4-Bis(2′-hydroxyethyl)amino-4-N-ethyl-2-nitro-benzol, 4-(2′-Hydroxyethyl)amino-3-nitro-toluol, 2-Nitro-4-(2′-hydroxyethyl)amino-anilin, 2,5-Bis (2′-hydroxyethyl)amino-nitrobenzol, 2-(2′-Hydroxyethyl)amino-4,6-dinitro-phenol, 1-Amino-4-(2′,3′-dihydroxypropyl)amino-2-nitro-5-chlor-benzol, 4-Bis(2′-hydroxyethyl)amino-1-(2′-hydroxyethyl)-amino-2-nitro-benzol, 1-Amino-2-nitro-4-bis(2′-hydroxyethyl)amino-benzol; Triphenylmethanfarbstoffe, wie zum Beispiel Basic Violet 1 (C. I. 42535); Azofarbstoffe, wie zum Beispiel Acid Brown 4 (C. I. 14805); Anthrachinonfarbstoffe, wie z. B. Disperse Blue 23 (C. I. 61545), Disperse Violet 4 (C. I. 61105), 1,4,5,8-Tetraamino-anthrachinon und 1,4-Diamino-antrachinon, wobei die Farbstoffe dieser Klassen je nach Art ihrer Substituenten sauren, nichtionogenen oder basischen Charakter haben können. Weitere geeignete, direkt auf das Haar aufziehende Farbstoffe sind beispielsweise in dem Buch von J. C. Johnson, "Hair Dyes", Noyes Data Corp., Park-Ridge (USA) (1973), Seiten 3 - 91 und 113 - 139 (ISBN:0-8155-0477-2) beschrieben.

Die Zubereitungsform des hier beschriebenen Haarfärbemittels auf der Basis von direkt auf das Haar aufziehenden Farbstoffen kann beispielsweise eine Lösung, insbesondere eine wäßrige oder wäßrig-alkoholische Lösung, sein. Bevorzugte Zubereitungsformen sind weiterhin eine Creme, ein Gel oder eine Emulsion, wobei sie auch im Gemisch mit einem Treibgas oder mittels einer Pumpe versprüht werden können.

Die Farbstoffe der allgemeinen Formel (I) sollen in diesem Haarfärbemittel ohne Oxidationsmittel-Zugabe in einer Konzentration von etwa 0,01 bis 2,0 Gewichtsprozent, vorzugsweise von 0,01 bis 1,0 Gewichtsprozent, enthalten sein. Der Gesamtgehalt an den direkt auf das Haar aufziehenden Farbstoffen liegt in den Grenzen von etwa 0,01 bis 3,0 Gewichtsprozent.

Der pH-Wert dieses Färbemittels liegt im Bereich von 3 bis 10,5, insbesondere bei pH 7,5 bis 9,5, wobei die Einstellung des gewünschten alkalischen pH-Wertes vorzugsweise mit Ammoniak erfolgt, jedoch auch mit organischen Aminen, wie beispielsweise Monoethanolamin oder Triethanolamin, vorgenommen werden kann.

Seine Anwendung erfolgt in üblicher Weise durch Aufbringen einer für die Haarfärbung ausreichenden Menge des Mittels auf die Haare, mit denen es eine Zeitlang, etwa 5 bis 30 Minuten, in Berührung bleibt. Anschließend wird das Haar mit Wasser, gegebenenfalls noch mit der wäßrigen Lösung einer schwachen organischen Säure, gespült und sodann getrocknet. Als schwache organische Säure können beispielsweise Essigsäure, Zitronensäure, Weinsäure und ähnliche Verwendung finden.

Das vorstehend beschriebene Haarfärbemittel ohne Oxidationsmittel-Zugabe kann selbstverständlich auch kosmetische Polymerisate enthalten, wodurch gleichzeitig mit der Färbung eine Festigung der Haare erreicht wird. Solche Mittel werden im allgemeinen als Tönungsfestiger oder Farbfestiger bezeichnet.

Von den für diesen Zweck in der Kosmetik bekannten synthetischen Polymeren seien beispielsweise Polyvinylpyrrolidon, Polyvinylacetat, Polyvinylalkohol oder Polyacrylverbindungen wie Polyacrylsäure und Polymethacrylsäurepolymerisate, basische Polymerisate von Estern aus diesen Verbindungen und Aminoalkoholen oder deren Salze oder Quaternierungsprodukte, Polyacrylnitril, Polyvinyllactame sowie Copolymerisate aus derartigen Verbindungen wie zum Beispiel Polyvinylpyrrolidon-Vinylacetat und dergleichen erwähnt.

Auch natürliche Polymere, wie Chitosan (entacetyliertes Chitin) oder Chitosanderivate, können für den genannten Zweck eingesetzt werden.

Die Polymerisate sind in diesem Mittel in der für solche Mittel üblichen Menge von etwa 1 bis 5 Gewichtsprozent enthalten. Der pH-Wert des Mittels liegt im Bereich von etwa 6,0 bis 9,0.

Die Anwendung dieses Haarfärbemittels mit zusätzlicher Festigung erfolgt in bekannter und üblicher Weise durch Befeuchten des Haares mit dem Festiger, Festlegen (Einlegen) des Haares zur Frisur und anschließende Trocknung.

Selbstverständlich kann das vorstehend beschriebene Haarfärbemittel ohne Oxidationsmittel-Zugabe gegebenenfalls weitere, für Haarfärbemittel übliche Zusätze, wie zum Beispiel Pflegestoffe, Netzmittel, Verdicker, Weichmacher, Konservierungsstoffe und Parfümöle sowie auch andere, nachstehend für Oxidationshaarfärbemittel aufgeführte übliche Zusätze enthalten.

Zum Gegenstand der vorliegenden Erfindung gehört auch, wie eingangs erwähnt, ein Haarfärbemittel, bei dem die Zugabe eines Oxidationsmittels erforderlich ist. Es enthält außer den Farbstoffen gemäß der angegebenen Formel (I) und gegebenenfalls bekannten, direkt auf das Haar aufziehenden Farbstoffen noch zusätzliche, bekannte Oxidationsfarbstoffe, die einer oxidation Entwicklung bedürfen.

Bei diesen Oxidationsfarbstoffen handelt es sich hauptsächlich um aromatische p-Diamine und p-Aminophenole, wie beispielsweise p-Toluylendiamin, p-Phenylendiamin, p-Aminophenol und ähnliche Verbindungen, die zum Zwecke der Nuancierung der Färbungen mit sogenannten Modifiern, wie zum Beispiel m-Phenylendiamin, Resorcin, m-Aminophenol und anderen, kombiniert werden.

Derartige zur Haarfärbung bekannte und übliche Oxidationsfarbstoffe sind unter anderem in dem Buch

von E.Sagarin, "Cosmetics" Science and Technology (1957), Interscience Publishers Inc., New York, Seiten 503 ff. sowie in dem Buch von H. Janistyn, "Handbuch der Kosmetika und Riechstoffe" (1973), Seiten 338 ff., beschrieben.

Mit Mischungen aus diesen Oxidationsfarbstoffen und den Farbstoffen gemäß der angegebenen Formel (I) lassen sich sehr gut natürliche Blond- und Brauntöne, aber auch modische Nuancen herstellen.

Die Farbstoffe gemäß der Formel (I) sind in diesem Färbemittel mit Oxidationsmittel-Zugabe in einer Konzentration von etwa 0,01 bis 2,0 Gewichtsprozent, vorzugsweise 0,01 bis 1,0 Gewichtsprozent, enthalten. Der Gesamtgehalt an Farbstoffen in diesem Färbemittel beträgt etwa 0,1 bis 5,0 Gewichtsprozent.

Oxidationshaarfärbemittel sind im allgemeinen alkalisch eingestellt, vorzugsweise auf pH-Werte von etwa 8,0 bis 11,5, wobei die Einstellung überwiegend mit Ammoniak erfolgt. Es können dazu aber auch andere organische Amine, zum Beispiel Monoethanolamin oder Triethanolamin, verwendet werden. Als Oxidationsmittel zur Entwicklung der Haarfärbungen kommen hauptsächlich Hydrogenperoxid und dessen Additionsverbindungen in Betracht. Die Zubereitungsform dieses Haarfärbemittels kann die gleiche wie bei dem Haarfärbemittel ohne Oxidationsmittel-Zugabe sein. Vorzugsweise ist sie die einer Creme oder eines Gels.

Übliche Zusätze in Cremes, Emulsionen oder Gelen sind zum Beispiel Lösungsmittel wie Wasser, niedere aliphatische Alkohole, beispielsweise Ethanol, Propanol, Isopropanol, Glycerin oder Glykole, wie zum Beispiel Ethylenglykol und Propylenglykol, oder auch Glykolether, weiterhin Netzmittel oder Emulgatoren aus den Klassen der anionischen, kationischen, amphoteren oder nichtionogenen oberflächenaktiven Substanzen, wie zum Beispiel Fettalkoholsulfate, Fettalkoholethersulfate, Alkylsulfonate, Alkylbenzolsulfate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker, wie zum Beispiel höhere Fettalkohole, Bentonit, Stärke, Polyacrylsäure, Cellulosederivate wie zum Beispiel Carboxymethylcellulose, Alginate, Vaseline, Paraffinöl und Fettsäuren sowie außerdem Pflegestoffe, wie zum Beispiel Lanolinderivate, Cholesterin, Pantothensäure und Betain, weiterhin Parfümöle und Komplexbildner. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, während die Verdicker in einer Menge von etwa 0,1 bis 5 Gewichtsprozent in den Zubereitungen enthalten sein können.

Die Anwendung der genannten Zubereitungen, bei denen die Zugabe eines Oxidationsmittels erforderlich ist, erfolgt in bekannter Weise, indem man die Haarfärbemittel vor der Behandlung mit dem Oxidationsmittel vermischt und eine zur Färbung des Haares ausreichende Menge der Mischung, im allgemeinen etwa 50 bis 150 ml, auf das Haar aufträgt. Nach einer für die Haarfärbung ausreichenden Einwirkungszeit, die üblicherweise etwa 10 bis 45 Minuten beträgt, wird das Haar mit Wasser, gegebenenfalls anschließend mit der wäßrigen Lösung einer schwachen organischen Säure, wie zum Beispiel Zitronensäure oder Weinsäure, gespült und sodann getrocknet.

Hinsichtlich der färberischen Möglichkeiten bieten die erfindungsgemäßen Haarfärbemittel je nach Art und Zusammensetzung der Farbkomponenten eine breite Palette verschiedener Farbnuancen, die sich von natürlichen Farbtönen bis hin zu hochmodischen, leuchtenden Nuancen erstreckt. Hierbei werden die Färbemittel je nach Zusammensetzung entweder in Verbindung mit Hydrogenperoxid oder auch ohne Oxidationsmittel angewandt.

Die 2,6-Dinitro-phenolderivate der Formel (I) sind zum Teil bekannt. So ist zum Beispiel die Herstellung für folgende Verbindungen in der Literatur beschrieben:

| X = | Literatur |
|---|---|
| $CH_3$ | G.G.S. Dutton, T.I.Briggs, B.R.Brown und M.E.D. Hillman: Can. J. Chem. 31, 685 (1953). |
| $C_2H_5$ | G.G.S. Dutton, T.I.Briggs, B.R.Brown und M.E.D. Hillman: Can. J. Chem. 31, 837 (1953). |
| $C_3H_7$ | G.G.S. Dutton, T.I.Briggs, B.R.Brown und M.E.D. Hillman: Can. J. Chem. 31, 685, 837 (1953). |
| $C_4H_9$ | G.G.S. Dutton, T.I.Briggs, B.R.Brown und M.E.D. Hillman: Can. J. Chem. 31, 837, 1138 (1953). |
| $CF_3$ | Jagupolskii und Mospan: Ukr. Chim. Z. 21, 81 (1955); C.A. 1955, 8866. |
| Cl | U.Kohn und R.Kramer: Monatsh. 49, 154 (1923). |
| Br | E.Sakellarios: Chem. Ber. 55, 2846 (1922). |
| $OH_3$ | W.B.Shaw: J. Chem. Soc. 99, 1613 (1911). |
| $CH_2OH$ | US-PS 4661382. |

Die übrigen Verbindungen der allgemeinen Formel (I) lassen sich nach den dort beschriebenen Verfahren analog synthetisieren.

Die folgenden Beispiele sollen den Gegenstand der Anmeldung weiter erläutern, ohne ihn auf die Beispiele zu beschränken.

**Haarfärbebeispiele**

**Beispiel 1:** Flüssiges Haarfärbemittel

| | |
|---|---|
| 0,30 g | 2,6-Dinitro-4-methoxy-phenol |
| 2,00 g | Laurylalkohol-diglykolethersulfat-Natriumsalz (28 prozentige wäßrige Lösung) |
| 2,00 g | Ammoniak (25 prozentige wäßrige Lösung) |
| 95,70 g | Wasser |
| 100,00 g | |

Gebleichtes Naturhaar wird 20 Minuten lang bei Raumtemperatur mit der Lösung nach Beispiel 1 behandelt, danach mit Wasser gespült und anschließend getrocknet. Das Haar ist leuchtend orangerot gefärbt.

**Beispiel 2:** Farbfestiger

| | |
|---|---|
| 0,10 g | 2,6-Dinitro-4-trifluormethyl-phenol |
| 2,00 g | Polyvinylpyrrolidon |
| 0,10 g | Glycerin |
| 40,00 g | Isopropanol |
| 57,80 g | Wasser |
| 100,00 g | |

Weiße menschliche Haare werden mit der Farbfestigerlösung befeuchtet, auf Wickler gewickelt und getrocknet. Anschließend werden die Wickler entfernt und das Haar zur Frisur gekämmt. Das Haar ist leuchtend zitronengelb gefärbt und gefestigt.

**Beispiel 3:** Oxidationshaarfärbemittel in Cremeform

| | |
|---|---|
| 0,10 g | 4-Brom-2,6-dinitro-phenol |
| 0,20 g | p-Phenylendiamin |
| 0,15 g | Resorcin |
| 0,03 g | m-Aminophenol |
| 15,00 g | Cetylalkohol |
| 3,50 g | Laurylalkohol-diglykolethersulfat-Natriumsalz (28 prozentige, wäßrige Lösung) |
| 6,00 g | Ammoniak (25 prozentige wäßrige Lösung) |
| 75,02 g | Wasser |
| 100,00 g | |

50 g des vorstehenden Haarfärbemittels werden kurz vor der Anwendung mit 50 ml 6 prozentiger wäßriger Wasserstoffperoxidlösung gemischt. Das Gemisch wird anschließend auf graue menschliche Haare aufgetragen und 30 Minuten lang bei einer Temperatur von 40 Grad Celsius einwirken gelassen. Nach dem Spülen des Haares mit Wasser und dem anschließenden Trocknen hat es einen modischen Blondton angenommen.

**Beispiel 4:** Haarfärbemittel in Cremeform

| | |
|---|---|
| 0,050 g | 4-Chlor-2,6-dinitro-phenol |
| 0,250 g | 4-Ethyl-(2'-hydroxyethyl)amino-1-(2'-hydroxyethyl)amino-2-nitro-benzol |
| 0,020 g | 1-Amino-2-nitro-4-bis(2'-hydroxyethyl)amino-benzol |
| 0,025 g | Disperse Blue 23 (C.I. 61545) |
| 7,500 g | Cetylalkohol |
| 1,750 g | Laurylalkohol-diglykolethersulfat-Natriumsalz (28 prozentige wäßrige Lösung) |
| 0,100 g | p-Hydroxybenzoesäuremethylester |
| 0,200 g | Ammoniak (25 prozentige wäßrige Lösung) |
| 90,105 g | Wasser |
| 100,000 g | |

50 g des vorstehenden Haarfärbemittels werden auf weiße menschliche Haare aufgetragen und nach einer Einwirkzeit von 20 Minuten mit Wasser ausgespült. Das Haar wird anschließend getrocknet. Es ist in einem natürlichen, braunen Farbton gefärbt.

**Beispiel 5:** Flüssiges Haarfärbemittel

| | |
|---|---|
| 0,25 g | 2,6-Dinitro-4-methoxy-phenol |
| 0,10 g | 1-Amino-4-(2',3'-dihydroxypropyl)amino-2-nitro-5-chlor-benzol |
| 0,20 g | 4-Ethyl-(2'-hydroxyethyl)amino-1-(2'-hydroxyethyl)amino-2-nitro-benzol |
| 0,50 g | Hydroxyethylcellulose |
| 5,00 g | Laurylalkohol-diglykolethersulfat-Natriumsalz 28 prozentige wäßrige Lösung) |
| 10,00 g | Isopropanol |
| 10,00 g | Ammoniak (25 prozentige wäßrige Lösung) |
| 73,95 g | Wasser |
| 100,00 g | |

Gebleichtes Naturhaar wird 20 Minuten lang bei Raumtemperatur mit der Lösung nach Beispiel 5 behandelt. Nach der Spülung des Haares mit Wasser und der anschließenden Trocknung ist das Haar in einem modischen Beaujolais-Ton eingefärbt.

**Beispiel 6:** Flüssiges Haarfärbemittel

| | |
|---|---|
| 0,30 g | 4-Brom-2,6-dinitro-phenol |
| 0,05 g | 1,4-Bis(2'-hydroxyethyl)amino-2-nitro-benzol |
| 0,50 g | Hydroxyethylcellulose |
| 5,00 g | Laurylalkohol-diglykolethersulfat-Natrium-salz (28 prozentige wäßrige Lösung) |
| 10,00 g | Isopropanol |
| 10,00 g | Ammoniak (25 prozentige wäßrige Lösung) |
| 74,15 g | Wasser |
| 100,00 g | |

Das vorstehende Färbemittel wird auf gebleichte Naturhaare 30 Minuten lang bei 30 Grad Celsius einwirken gelassen. Nach der Spülung des Haares mit Wasser und der anschließenden Trocknung ist das Haar in einem modischen hellen Blondton gefärbt.

**Patentansprüche**

1. Verwendung eines 2,6-Dinitro-phenolderivats der allgemeinen Formel (I),

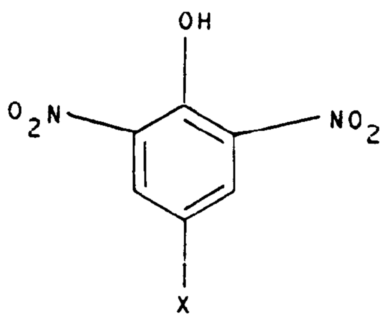

(I),

wobei X die Bedeutung ($C_1$-$C_4$)-Alkyl, ($C_1$-$C_4$)-Monohydroxyalkyl, ($C_1$-$C_4$)-Perfluoralkyl, ($C_1$-$C_4$)-Alkoxy, ($C_2$-$C_4$)-Monohydroxyalkoxy, ($C_3$-$C_4$)-Dihydroxyalkoxy oder Halogen hat, als Farbstof in Haarfärbemitteln.

2. Verwendung eines 2,6-Dinitro-phenolderivats der allgemeinen Formel (II)

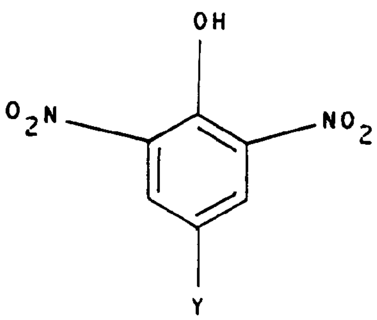

(II),

wobei Y die Bedeutung $CH_3$, $C(CH_3)_3$, $CF_3$, $CH_2OH$, $OCH_3$, Cl oder Br hat, als Farbstoff in Haarfärbemitteln.

3. Mittel zur Färbung von Haaren mit einem Farbstoffgehalt und für Haarfärbemittel üblichen Zusätzen dadurch gekennzeichnet, daß es 0,01 bis 2,0 Gewichtsprozent eines 2,6-Dinitro-phenolderivates der Formel (I) gemäß Anspruch 1 enthält.

4. Mittel nach Anspruch 3, dadurch gekennzeichnet, daß es mindestens einen bekannten direkt auf das Haar aufziehenden Farbstoff enthält.

5. Mittel nach Anspruch 4, dadurch gekennzeichnet, daß der bekannte, direkt auf das Haar aufziehende Farbstoff ausgewählt ist aus 2-Amino-4-nitro-phenol, Pikraminsäure, 2-(2'-Hydroxyethyl)amino-4,6-dinitrobenzol 1-(2'-Hydroxyethyl)amino-2-amino-4-nitro-benzol, 4-(2'-Ureidoethyl)amino-nitrobenzol, 4-(2',3'-Dihydroxypropyl)amino-3-nitro-trifluormethylbenzol, 4-(2'-Hydroxyethyl)amino-3-nitrotoluol, 2-Nitro-4-(2'-hydroxyethyl)amino-anilin, 1,4-Bis(2'-hydroxyethyl)amino-4-N-ethyl-2-nitro-benzol, 2,5-Bis(2'-hydroxyethyl)amino-nitrobenzol, 2-(2'-Hydroxyethyl)amino-4,6-dinitro-phenol, 1-Amino-4-(2',3'-dihydroxypropyl)amino-2-nitro-5-chlorbenzol, 4-Bis(2'-hydroxyethyl)amino-1-(2'-hydroxyethyl)amino-2-nitro-benzol, 1-Amino-2-nitro-4-bis(2'-hydroxyethyl)amino-benzol, Basic Violet 1 (C.I. 42535), Acid Brown 4 (C.I. 14805) und Disperse Violet 4 (C.I. 61105), Disperse Blue 23 (C.I. 61545), 1, 4, 5, 8-Tetraaminoanthrachinon und 1,4-Diamino-anthrachinon.

6. Mittel nach einem der Ansprüche 3 bis 5, dadurch gekennzeichnet, daß es in form eines Haarfärbemittels mit zusätzlicher Haarfestigung vorliegt und mindestens ein in der Kosmetik üblicherweise verwendetes synthetisches oder natürliches Polymer enthält.

7. Mittel nach Anspruch 6, dadurch gekennzeichnet, daß das synthetische oder das natürliche Polymer ausgewählt ist aus Chitosan oder dessen Derivaten, Polyvinylpyrrolidon, Polyvinylacetet, Polyvinylalkohol, Polyacrylsäure, Polymethacrylsäure, Polyacrylnitril, Polyvinylactam oder Copolymerisaten aus diesen Verbindungen.

8. Mittel nach Anspruch 3, dadurch gekennzeichnet, daß es mindestens einen bekannten Oxidationshaarfarbstoff enthält.

9. Mittel nach Anspruch 8, dadurch gekennzeichnet, daß der bekannte Oxidationshaarfarbstoff ausgewählt ist aus p-Toluylendiamin, p-Phenylendiamin, p-Aminophenol, m-Phenylendiamin, Resorcin und m-Aminophenol.

## Claims

1. Application of a 2, 6-dinitro-phenol derivative having the. general formula (I),

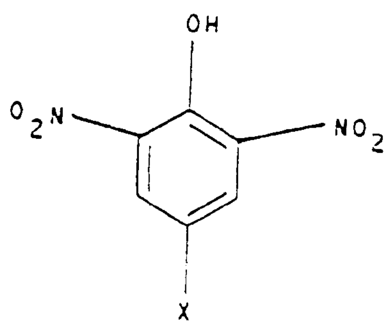

(I)

wherein X represents ($C_1$-$C_4$)-alkyl, ($C_1$-$C_4$)-monohydroxyalkyl, ($C_1$-$C_4$)-perfluoroalkyl, ($C_1$-$C_4$)-alkyoxy, ($C_2$-$C_4$)-monohydroxyalkoxy, ($C_3$-$C_4$)-dihydroxyalkoxy or halogen, as a dyestuff in hair colorants.

2. Application of a 2,6-dinitro-phenol derivative having the. general formula (II),

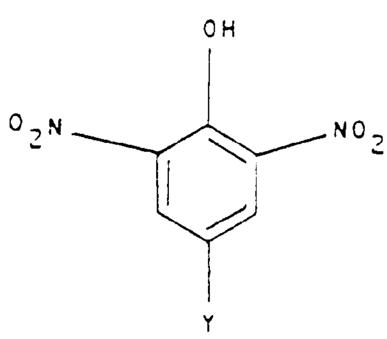

(II)

wherein Y represents $CH_3$, $C(CH_3)_3$, $CF_3$, $CH_2OH$, $OCH_3$, Cl or Br, as a dyestuff in hair colorants.

3. Agent for colouring hair having a content of dyestuff and additives usual for hair colorants, characterised in that it contains 0.01 to 2.0 weight percent of a 2,6-dinitro-phenol derivative of formula (I) according to claim 1.

4. Agent according to Claim 3, characterised in that it contains at least one known dyestuff applied directly to the hair.

5. Agent according to Claim 4, characterised in that the known dyestuff applied directly to the hair is selected from 2-amino-4-nitro-phenol, picraminic acid, 2-(2′-hydroxyethyl)amino-4,6-dinitro-benzene, 1-(2′-hydroxyethyl)amino-2-amino-4-nitro-benzene, 4-(2′-ureidoethyl)amino-nitrobenzene, 4-(2′,3′-dihydroxypropyl) amino-3-nitro-trifluoromethylbenzene, 4-(2′-hydroxyethyl)amino-3-nitrotoluene, 2-nitro-4-(2′-hydroxyethyl) amino-aniline, 1,4-bis(2′-hydroxyethyl)amino-4-N-ethyl-2-nitro-benzene, 2,5-bis(2′-hydroxyethyl)-amino-nitrobenzene, 2-(2′-hydroxyethyl)amino-4,6-dinitro-phenol, 1-amino-4-(2′,3′-dihydroxypropyl)amino-2-nitro-5-chlorobenzene, 4-bis(2′-hydroxyethyl)amino-1-(2′-hydroxyethyl)amino-2-nitro-benzene, 1-amino-2-nitro-4-bis (2′-hydroxyethyl)amino-benzene, Basic Violet 1 (C.I. 42535), Acid Brown 4 (C.I. 14805) and Disperse Violet 4 (C.I. 61105), Disperse Blue 23 (C.I. 61545), 1, 4, 5, 8-tetraaminoanthraquinone and 1, 4-diaminoanthraquinone.

6. Agent according to one of Claims 3 to 5, characterised in that it is in the form of a hair colorant with additional hair fixing ability and contains at least one synthetic or natural polymer usually used in cosmetics.

7. Agent according to Claim 6, characterised in that the synthetic or natural polymer is selected from chitosan or the derivatives thereof, polyvinylpyrrolidone, polyvinylacetate, polyvinylalcohol, polyacrylic acid, polymethacrylic acid, polyacrylnitryl, polyvinyl lactam or copolymers of these compounds.

8. Agent according to Claim 3, characterised in that it contains at least one known oxidising hair colorant.

9. Agent according to Claim 8, characterised in that the known oxidising hair colorant is selected from p-toluylenediamine, p-phenylenediamine, p-aminophenol, m-phenylenediamine, resorcinol and m-aminophenol.

## Revendications

1. Utilisation d'un dérivé 2,6-dinitro-phénol de formule générale (I),

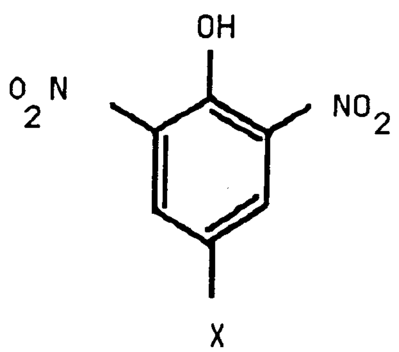

(I)

dans lequel X est un ($C_1$-$C_4$)-alkyle, un ($C_1$-$C_4$)-monohydroxyalkyle, un ($C_1$-$C_4$)-perfluoralkyle, un ($C_1$-$C_4$)-alcoxy, un ($C_2$-$C_4$)-monohydroxyalkoxy, un ($C_3$-$C_4$)-dihydroxyalkoxy ou un halogène, en tant que colorant dans des agents de coloration des cheveux.

2. Utilisation d'un dérivé 2,6-dinitro-phénol de formule générale (II),

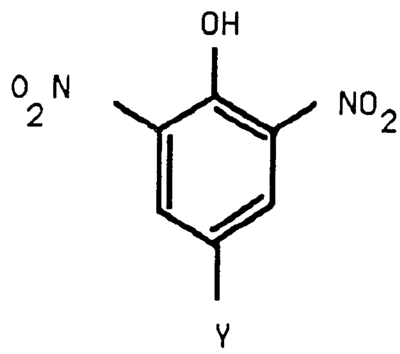

(II)

dans lequel Y est $CH_3$, $C(CH_3)_3$, $CF_3$, $CH_2OH$, $OCH_3$, Cl ou Br, en tant que colorant dans des agents de coloration des cheveux.

3. Agent de coloration des cheveux présentant une teneur en colorant et des additifs usuels comme agents de coloration des cheveux, caractérisé en ce qu'il contient de 0,01 à 2,0 pourcents en poids d'un dérivé 2,6-dinitrophénol de la formule (I) selon la revendication 1.

4. Agent selon la revendication 3, caractérisé en ce qu'il contient au moins un colorant connu nourrissant directement les cheveux.

5. Agent selon la revendication 4, caractérisé en ce que le colorant connu, nourrissant directement les cheveux, qui est sélectionné est sélectionné parmi les produits suivants : 2-amino-4-nitro-phénol, acide picramique, 2-(2'-hydroxyéthyl)amino-4,6-dinitro-benzène, 1-(2'-hydroxyéthyl) amino-2-amino-4-nitro-benzène, 4-(2'-uréidoéthyl)aminonitrobenzène, 4-(2',3'-dihydroxypropyl) amino-3-nitrotrifluorméthylbenzène, 4-(2'-hydroxyéthyl) amino-3-nitrotoluène, 2-nitro-4-(2'-hydroxyéthyl) amino-aniline, 1,4bis(2'-hydroxyéthyl) amino-4N-éthyl-2-nitro-benzène, 2,5bis(2'-hydroxyéthyl) amino-nitrobenzène, 2-(2'-hydroxyéthyl) amino-4,6-dinitrophénol, 1-amino-4-(2',3'-dihydroxypropyl) amino-2-nitro-5-chlorbenzène, 4bis-(2'-hydroxyéthyl) amino-1 -(2'-hydroxyéthyl)amino-2-nitrobenzène, 1-amino-2-nitro-4-bis- (2'-hydroxyéthyl) aminobenzène, Basic violet 1 (C.I.42535), Acid brown 4 (C.I.14805) et disperse violet 4 (C.I.61105), disperse blue 23 (C.I.61545), 1, 4, 5, 8-tetraaminoanthrachinon et 1,4-diamino-antrachinon.

6. Agent selon l'une des revendications 3 à 5, caractérisé en ce qu'il se présente sous la forme d'un agent de coloration des cheveux avec une fixation supplémentaire des cheveux et contient au moins un polymère synthétique ou naturel utilisé usuellement en cosmétique.

7. Agent selon la revendication 6, caractérisé en ce que le polymère synthétique ou naturel est sélectionné parmi les produits suivants : chitosane ou ses dérivés, polyvinylpyrrolidon, acétate de polyvinyle, alcool de polyvinyle, acide polyacrilique, acide polyméthacrylique, polyacrylnitrile, polyvinyllactame ou copolymérisats de ces liaisons.

8. Agent selon la revendication 3, caractérisé en ce qu'il contient au moins un colorant par oxydation des cheveux connu.

9. Agent selon la revendication 8, caractérisé en ce que le colorant par oxydation des cheveux connu est sélectionné parmi les produits suivants : p-toluylènediamine, p-phénylènediamine, p-aminophénol, m-phénylènediamine, résorcine et m-aminophénol.